(19) Europäisches Patentamt / European Patent Office / Office européen des brevets

(11) **EP 2 714 917 B1**

(12) **FASCICULE DE BREVET EUROPEEN**

(45) Date de publication et mention de la délivrance du brevet:
**14.10.2020 Bulletin 2020/42**

(21) Numéro de dépôt: **12731062.1**

(22) Date de dépôt: **25.05.2012**

(51) Int Cl.:
*C12P 5/02* *(2006.01)*      *C11B 1/02* *(2006.01)*
*C12P 5/00* *(2006.01)*

(86) Numéro de dépôt international:
**PCT/FR2012/051175**

(87) Numéro de publication internationale:
**WO 2012/164211 (06.12.2012 Gazette 2012/49)**

(54) **PROCÉDÉ D'EXTRACTION DU SQUALÈNE À PARTIR DE MICROALGUES**

VERFAHREN ZUR EXTRAKTION VON SQUALEN AUS MIKROALGEN

METHOD FOR EXTRACTING SQUALENE FROM MICROALGAE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.05.2011 FR 1154623**

(43) Date de publication de la demande:
**09.04.2014 Bulletin 2014/15**

(73) Titulaire: **Roquette Frères**
**62136 Lestrem (FR)**

(72) Inventeur: **PATINIER, Samuel**
**F-59000 Lille (FR)**

(74) Mandataire: **Plasseraud IP**
**66, rue de la Chaussée d'Antin**
**75440 Paris Cedex 09 (FR)**

(56) Documents cités:
EP-A1- 0 113 165      WO-A1-2010/096002
WO-A2-03/092628      WO-A2-2008/151149
WO-A2-2013/156720    FR-A1- 2 989 373
US-A1- 2008 160 593  US-A1- 2011 086 386

- CHEN G ET AL: "Optimization of nitrogen source for enhanced production of squalene from thraustochytrid Aurantiochytrium sp", NEW BIOTECHNOLOGY, ELSEVIER BV, NL, vol. 27, no. 4, 30 septembre 2010 (2010-09-30), pages 382-389, XP027171921, ISSN: 1871-6784 [extrait le 2010-04-20] cité dans la demande

**Description**

La présente invention se rapporte à un procédé d'extraction optimisée de squalène sans solvant organique à partir de microalgues de la famille des Thraustochytriales sp.

On entend au sens de l'invention par « microalgues de la famille des Thraustochytriales sp. » des microalgues appartenant aux espèces *Schizochytrium sp., Aurantiochytrium sp.* et *Thraustochytrium sp.*

Le squalène est un triterpène, isoprénoïde à trente atomes de carbone et cinquante atomes d'hydrogène, de formule : 2,6,10,15,19,23-Hexaméthyl-2,6,10,14,18,22-tétracosahexène.

C'est un lipide naturellement produit par tous les organismes supérieurs y compris chez l'Homme (retrouvé dans le sébum). Le squalène est en effet un intermédiaire essentiel dans la biosynthèse du cholestérol, des hormones stéroïdes et de la vitamine D (une enzyme des voies métaboliques du cholestérol, la squalène monooxygénase, en oxydant l'une des extrémités de la molécule de squalène, va induire sa cyclisation et conduire au lanostérol, lequel sera transformé en cholestérol et en autres stéroïdes).

En industrie, le squalène est surtout utilisé dans les domaines alimentaire, cosmétique et pharmaceutique.

Comme complément alimentaire, le squalène est habituellement formulé en capsules ou en huiles.

Dans le domaine cosmétique, cette molécule peut être utilisée comme antioxydant, antistatique et émollient dans les crèmes hydratantes, pénétrant rapidement la peau sans laisser de traces ou de sensations grasses, et se mélangeant bien avec d'autres huiles et vitamines.

Dans ce domaine, notons qu'étant donné la très grande instabilité du squalène (6 insaturations), c'est la forme saturée squalane (obtenue par hydrogénation), meilleur antioxydant que le squalène, que l'on trouve sur le marché, et à niveau de pureté en général très élevé (99%).

Les études toxicologiques ont montré qu'aux concentrations utilisées dans les cosmétiques, le squalène et le squalane ne présentent pas de toxicité, et ne sont pas irritants ou sensibilisants pour la peau humaine.

Dans le domaine pharmaceutique, le squalène est utilisé comme adjuvants aux vaccins.

Ces adjuvants sont des substances qui stimulent le système immunitaire et augmentent la réponse au vaccin.

Le niveau de pureté du squalène est essentiel dans ce domaine d'applications.

En effet, s'il est pris par voie orale, le squalène est considéré comme totalement sûr ; mais c'est la voie injectable qui fait l'objet de controverses.

En effet, dans le domaine médical, le risque de préjudice pour un receveur humain peut être accru dans les situations où le squalène est contaminé par des impuretés, car, par définition, cet adjuvant peut induire une forte réponse immunitaire également contre ses propres impuretés.

Il est donc indispensable de disposer de squalène de haute qualité, exempt d'impuretés (traces de métaux, notamment de mercure, et d'autres toxines).

Un certain nombre de voies de production et d'extraction du squalène sont proposées dans la littérature.

C'est un composé que l'on trouve souvent stocké dans le foie des poissons cartilagineux tels que les requins des profondeurs (d'où son nom).

Il est donc une des causes de leur surpêche, le requin étant déjà chassé pour ses ailerons. Les foies de requin sont ainsi désormais vendus pour produire des gélules qualifiées de "bonnes pour la santé".

Cependant, si le squalène commercialisé est ainsi principalement extrait de foies de requins, il n'est pas exempt de problème sanitaire.

En effet, les requins peuvent être infectés par des pathogènes pouvant produire des substances nuisibles pour l'homme. En outre, le foie des requins, organe d'élimination et de purification de l'organisme, peut contenir des toxines telles que la carchatoxine qui est néfaste pour l'homme.

Ces préoccupations environnementales (forte régression des requins) et sanitaires (le foie des poissons stocke aussi des toxines préoccupantes pour la santé) ont motivé son extraction à partir de végétaux.

Il est ainsi possible de l'isoler de l'huile d'olive, l'huile de palme, et dans d'autres huiles céréalières ou provenant de l'amarante, des semences, du son de riz, de germes de blé.

Cependant, l'inconvénient majeur est ici que le squalène est extrait en très faibles quantités, de l'ordre de 0,1 à 0,7 % en poids.

En première alternative à ces procédés d'extraction à partir de foies de requins ou de végétaux, souvent rendus coûteux par la mise en oeuvre de procédés d'enrichissement et de purification importants, ont été proposés de premiers procédés de production de squalène à partir de microorganismes : levures naturelles ou levures recombinantes, notamment de type *Saccharomyces.*

C'est ainsi que Saccharomyces cerevisiae est connue pour sa capacité à produire du squalène, cependant en très faible quantité : de l'ordre de 0,041 mg/g de biomasse (BHATTACHARJEE, P. et al, 2001, dans World J. Microb. Biotechnol., 17, pp 811-816).

L'optimisation de ces capacités de production a donc été travaillée, par le biais de la recombinaison génétique. Cependant, comme le présente la demande de brevet WO 2010/023551 pour le domaine médical (production de squalène d'une pureté supérieure à 97 % comme adjuvant de vaccins), cette première alternative n'est industrialisable que si l'on peut disposer de levures recombinantes hyperproductrices de squalène (à plus de 15 % en poids de cellules sèches).

Or, l'obtention de ces cellules recombinantes nécessite la mise en oeuvre de nombreuses étapes lourdes, longues et complexes d'ingénierie métabolique, par la mise en oeuvre d'outils de biologie moléculaire, conduisant à la stimulation des voies de biosynthèse du squalène et à l'inhibition des voies du catabolisme du squalène.

En deuxième alternative aux procédés d'extraction à partir de foies de requins ou de végétaux, ont été proposés des procédés prometteurs de production de squalène à partir de microalgues de la famille des Thraustochytriales (comprenant les genres *Thraustochytrium, Aurantiochytrium* et *Schizochytrium*), plus particulièrement *Schizochytrium mangrovei* ou *Schizochytrium limacinum.*

Ces microalgues produisent du squalène en conditions hétérotrophiques (absence de lumière ; apport de glucose comme source carbonée), et peuvent donc être manipulées aisément par l'homme du métier du domaine de la fermentation de microorganismes.

Ces procédés offrent donc, par le biais de conditions de fermentation contrôlées, des qualités de squalène dont la purification est aisément concevable pour répondre aux besoins alimentaires, cosmétiques et médicales.

Chez ces microalgues de la famille des Thraustochytriales, le squalène est cependant le coproduit d'autres composés lipidiques d'intérêt, tel l'acide docosahexaénoïque (ou DHA), acide gras polyinsaturé de la famille des $\omega$3.

Il apparaît ainsi que le squalène est surtout décrit comme l'un des composants de la fraction insaponifiable des huiles commerciales de DHA (à côté des caroténoïdes et des stérols).

A titre de comparaison, la souche de *Schizochytrium mangrovei* FB1 produit du DHA à raison de 6,2 % en poids sec de cellules, pour 0,017 % de squalène.

De ce fait, ces microorganismes qui produisent naturellement du squalène le font en faibles quantités :

- de l'ordre de 0,1 mg/g de biomasse, pour Thraustochytrid ACEM 6063 (cf. LEWIS et al, Mar. Biotechnol., 2001, pp 439-447),
- de l'ordre de 0,162 mg/g de biomasse, pour *Schizochytrium mangrovei* FB1 (cf. YUE JIANG et al, J. Agric. Food Chem., 2004, 52, pp 1196-1200)

Pour augmenter ces productions, il est alors apparu indispensable d'optimiser les conditions de fermentation.

Cependant, malgré tous les efforts déployés, ces valeurs restent inférieures à celles de référence pour l'huile d'olive (de l'ordre de 4,24 mg/g).

Au mieux, ces productions optimisées conduisent à produire de l'ordre de :

- 1 mg à 1,2 mg de squalène par g de biomasse de Thraustochytride ACEM 6063 (cf. QIAN Li et al, J. Agric. Food Chem., 2009, 57, 4267-4272 ou LEWIS et al, dans Mar. Biotechnol., 2001, 3, 439-447).
- 0,72 mg de squalène par g de biomasse de Schizochytrium (cf. G. CHEN et al, New Biotechnology, 2010, 27-4, pp 382-389).
- 0,53 mg de squalène par g de biomasse d'*Aurantiochytrium mangrovei* FB3l (cf. K. W. FAN et al, World J. Microbiol. Biotechnol., 2010, 26-3, pp 1303-1309)
- 1,17 $\pm$ 0,6 mg de squalène par g de biomasse de *Schizochytrium mangrovei* (cf. C-J YUE et Y. JIANG, Process Biochemisty, 2009, 44, 923-927).

La société demanderesse a contribué elle aussi à améliorer encore la production de squalène par des microalgues de la famille des Thraustochytriales sp. en proposant un procédé permettant de produire le squalène à un niveau encore jamais atteint dans la littérature du domaine, i.e. d'au moins 8 g de squalène pour 100 g de biomasse (comme il sera exemplifié ci-après).

A l'échelle du laboratoire, les méthodes d'extraction du squalène à partir de la biomasse issue des milieux de fermentation sont classiquement des méthodes utilisant des solvants organiques :

- dans YUE JIANG et al, J. Agric. Food Chem., 2004, 52, 1196 - 1200, il est décrit un procédé dans lequel les lipides sont solubilisées dans du méthanol / acétone (7:3 v/v) puis lavés dans du chloroforme / méthanol (2:1 v/v) ;
- dans C-J YUE et Y. JIANG, Process Biochemisty, 2009, 44, 923-927, l'extraction du squalène et du cholestérol est réalisée à l'hexane après saponification préalable à l'éthanol des cellules lyophilisées ;
- dans G. CHEN et al, dans New Biotechnology, 2010, 27-4, pp 382-389, l'extraction du squalène est réalisée à l'hexane après saponification par KOH (10 % p/v) - éthanol (75 % v/v) des cellules lyophilisées ;
- dans LEWIS et al, Mar. Biotechnol., 2001, 439-447, il est d'abord procédé à l'extraction des lipides totaux à partir des cellules lyophilisées à l'aide d'un mélange ternaire chloroforme/méthanol/eau (1:2:0,8 v/v/v), puis, pour obtenir les lipides insaponifiables, une partie de ces lipides totaux est traitée avec une solution à 5 % de KOH dans du méthanol/eau (4 1 p/v) suivi d'une extraction proprement dite des lipides insaponifiables neutres à l'hexane-chloroforme (4:1 v/v) ;

A plus grande échelle, pour éviter l'utilisation de solvants néfastes pour l'homme et l'environnement, d'autres solutions ont été proposées.

A titre anecdotique, dans le brevet KR 2008/0017960, il est proposé par exemple de placer le milieu contenant du squalène dans une solution de cyclodextrines de manière à obtenir des complexes cyclodextrines/squalène, puis ajouter un agent de coagulation tel le $CaCl_2$, le CaSO4, le $MgCl_2$ ou le $MgSO_4$ afin de faciliter sa séparation dudit milieu. Il faut cependant encore le dé-complexifier le squalène afin de l'isoler en tant que tel.

Mais en fait, deux technologies sont principalement décrites :

- les procédés d'extraction au $CO_2$ supercritique ;
- les procédés d'extraction en l'absence de solvants organiques.

La première alternative aux procédés d'extraction au chloroforme ou à l'hexane est donc le $CO_2$ supercritique.

Cette technologie est bien adaptée à l'extraction de composés non polaires présentant un poids moléculaire de moins de 500 Daltons (celui du squalène est légèrement en dessous de 400 Da).

Le squalène est soluble dans le $CO_2$ supercritique à une pression comprise entre 100 et 250 bar).

De nombreux travaux d'extraction avec cette technologie ont été entrepris sur *Botryococcus braunii*, *Scenedesmus obliquus* ou *Torulaspora delbrueckii.*

Le $CO_2$ supercritique est par ailleurs ainsi aussi bien utilisé pour la lyse cellulaire que pour l'isolement du squalène.

Cependant, il est recommandé de lyophiliser les cellules avant d'en extraire les lipides, ce qui nécessite de nombreux travaux complémentaires pour permettre d'adapter les techniques au type de microorganisme.

Par ailleurs, ces conditions sont difficilement transposables à l'échelle industrielle à des coûts attractifs.

La seconde alternative technologique est celle de l'extraction des lipides en l'absence de solvants organiques.

Les enseignements tirés des nombreux articles et documents de BENEMANN et OSWALD, ou des brevets par exemple EP 1.252.324 et EP 1.305.440 décrivent cette approche, cependant sans qu'aucun ne précise les conditions optimisées d'extraction du squalène.

Dans leur article de 1996 intitulé Systems and Economic Analysis of Microalgae Ponds for Conversion of CO2 to Biomass. Report prepared for the Pittsburgh Energy Technology Center under Grant No. DE-FG22-93PC93204, BENEMANN, J. & OSWALD, W. enseigne que la centrifugation peut être utilisée non seulement pour concentrer la biomasse, mais aussi pour extraire simultanément les lipides des algues dans une phase huileuse.

Cette séparation est basée sur la différence relativement importante de densité de l'eau, des lipides des algues et des autres constituants de la biomasse.

OSWALD & BENNEMAN la décrive surtout dans le cadre d'un processus d'extraction du bêta-carotène de la biomasse algale floculées par un procédé d'extraction de l'huile chaude.

Ainsi, les étapes de récolte et de traitement se recoupent, avec les étapes communes de floculation et de centrifugation.

Dans le brevet EP 1.252.324, il est rapporté que le cassage de la biomasse microbienne humide pour libérer les lipides intracellulaires, le traitement du lysat cellulaire par un procédé permettant de produire un « mélange séparé en phase » comprenant une couche lourde et une couche légère, la séparation par gravité de la couche lourde de la couche légère qui contient les lipides, puis le cassage de l'émulsion eau / lipides dans ladite phase légère pour obtenir les lipides.

Il est important de noter que l'état d'émulsion empêche la récupération de lipides purs. Il est donc nécessaire d'avoir recours à un procédé de lavage de l'émulsion à l'aide d'une solution de lavage qui peut être de l'eau, de l'alcool et/ou de l'acétone jusqu'à ce que les lipides deviennent « sensiblement » non émulsifiés. Il est cependant préconisé de ne pas utiliser plus de 5 % de solvant organique non polaire.

On comprend également que l'interface huile / eau de l'émulsion est stabilisée par les débris cellulaires. C'est la raison pour laquelle le chauffage du milieu de fermentation avant ou pendant l'étape de cassage des cellules, ou l'addition d'une base au milieu de fermentation pendant l'étape de cassage cellulaire contribue à réduire la formation de l'émulsion, car ce traitement thermique (au moins 50°C) ou alcalin dénature les protéines et solubilise les matériaux organiques.

Ce procédé est dit permettant l'extraction de tous types de lipides : phospholipides ; acides gras libres ; esters d'acides gras, incluant les triglycérides of d'acides gras ; les stérols ; les pigments (e.g. caroténoïdes et oxycaroténoïdes) et autres lipides, et composés associés aux lipides tels que les phytostérols, l'ergothionine, l'acide lipoïque et les antioxydants incluant le bêta-carotène, les tocotrienols et tocopherol.

Les lipides et les composés associés aux lipides préférés sont ici le cholestérol, les phytostérols, desmostérols, tocotrienols, tocopherols, ubiquinones, caroténoïdes et xanthophylles tels que le beta-carotène, lutéine, lycopène, astaxanthine, zéaxanthine, canthaxanthine, et acides gras tels que les acides linoléiques conjugués, et les acides gras polyinsaturés de type omega-3 et omega-6 tels que les acides eicosapentaénoïque, docosapentaénoïque, docosahexaénoïque, arachidonique, stéaridonique, dihomogammalinolénique et gamma-linolénique.

Le squalène n'est pas envisagé en tant que tel, ni aucun procédé spécifique de lyse cellulaire ou de conditions de conduite de la centrifugation n'est explicitement fourni.

Quant au brevet EP 1.305.440, il est surtout dédié à l'extraction de l'acide arachidonique produit par *Mortierella alpina.*

**Soucieuse de mettre au point un procédé d'extraction du squalène plus efficace que ceux décrits dans l'état de la technique,** la société Demanderesse a développé ses propres recherches sur l'optimisation des conditions d'extraction sans solvant organique de ce composé à partir des milieux de fermentation des microalgues de la famille des Thraustochytriales sp.

L'invention concerne donc un procédé d'extraction sans solvant organique de squalène produit par fermentation de microalgues appartenant à la famille des Thraustochytriales sp., caractérisé en ce qu'il comprend les étapes suivantes :

1) préparer une biomasse de microalgues appartenant à la famille des Thraustochytriales de manière à réduire la concentration en solubles interstitiels et atteindre ainsi une pureté supérieure à 95 % exprimée en poids sec de biomasse sur poids sec total du milieu de fermentation,

2) traiter la biomasse ainsi obtenue à l'aide d'une enzyme de type protéase choisie dans le groupe des protéases neutres ou basiques, par exemple l'alcalase, de manière à rompre la paroi cellulaire desdites microalgues tout en prévenant la formation de l'émulsion produite par ledit traitement enzymatique,

3) centrifuger le mélange réactionnel ainsi obtenu afin de séparer l'huile de la phase aqueuse, et

4) récupérer l'huile brute enrichie en squalène ainsi produite.

La première étape du procédé conforme à l'invention consiste à préparer une biomasse de microalgues appartenant à la famille des Thraustochytriales de manière à réduire la concentration en solubles interstitiels et atteindre ainsi une pureté supérieure à 95 % exprimée en poids sec de biomasse sur poids sec total du milieu de fermentation.

Au sens de l'invention, on entend par « solubles interstitiels » tous les contaminants organiques solubles du milieu de fermentation, e.g. les composés hydrosolubles tels les sels, le glucose résiduel, les protéines et peptides...

Comme microalgues appartenant à la famille des Thraustochytriales, les souches commercialisées suivantes ont été testées :

- *Schizochytrium sp.* référencée ATCC 20888,
- *Aurantiochytrium sp.* référencée ATCC PRA 276,

Par ailleurs, la société Demanderesse dispose également de sa propre souche de production, une *Schizochytrium sp.* déposée le 14 avril 2011 en France auprès de la Collection Nationale de Cultures de Microorganismes de l'Institut Pasteur sous le n°CNCM I-4469 et également déposée en Chine auprès du CHINA CENTER FOR TYPE CULTURE COLLECTION de l'université de Wuhan, Wuhan 430072, P.R. China sous le n° M 209118.

La culture est réalisée en conditions hétérotrophiques. Généralement, l'étape de culture comprend une étape de préculture, pour revivifier la souche, puis une étape de culture ou de fermentation proprement dite. Cette dernière étape correspond à l'étape de production des composés lipidiques d'intérêt.

Les conditions de culture de ces microalgues sont bien connues dans le domaine. Par exemple dans l'article de G. CHEN dans New Biotechnology, 2010, 27-4, pp 382-389, on trouve un procédé comprenant les étapes successives suivantes :

- partir de la souche maintenue sur milieu nutritif gélosée comprenant du glucose, du glutamate monosodique, de l'extrait de levures et des oligoéléments divers,
- réaliser une préculture en Erlenmeyers sur agitateur orbital, à un pH de 6, à une température de 25°C afin d'obtenir une biomasse revivifiée,
- ensemencer une autre série d'Erlenmeyers de production, avec le même milieu de culture que celui utilisé en préculture, avec d'environ 0,5 % (v/v) de la biomasse obtenue à l'étape précédente, et maintenir la température à 25°C.

La préculture peut durer de préférence de 24 à 74 heures, de préférence environ 48 heures. La culture quant à elle peut durer de préférence de 60 à 150 heures.

La source carbonée nécessaire à la croissance de la microalgue est préférentiellement du glucose.

Quant à la nature de la source d'azote, la société Demanderesse a trouvé qu'il est possible de la choisir dans le groupe constitué des extraits de levure, de l'urée, du glutamate de sodium, du sulfate d'ammonium pris seuls ou en combinaison. De la même façon, il est possible de remplacer l'urée par du glutamate de sodium en tout ou partie, ou utiliser un mélange de glutamate de sodium et de sulfate d'ammonium.

Il est possible de préférer aux extraits de levure, classiquement mis en oeuvre dans les procédés de l'état de la technique, de l'urée complétée par un cocktail de vitamines, telle le cocktail BME commercialisé par la société SIGMA, utilisé à raison de 5 ml/l.

De préférence, les milieux de préculture comprennent des vitamines B1, B6 et B12.

Quant au pH du milieu de culture, comme il sera exemplifié ci-après, il sera maintenu entre 5,5 et 6,5, préférentiellement fixé à une valeur de 6. La régulation du pH peut se faire par tout moyen connu de l'homme du métier, par exemple par ajout d'acide sulfurique 2 N, puis avec de la soude 8 N.

Enfin, le taux d'oxygène dissous peut être régulé à une valeur comprise entre 20 et 0 %, de préférence maintenu à 5 % pendant une durée initiale de 24 et 48 heures, de préférence 36 heures, avant d'être laissée à 0 %. Quant au transfert d'oxygène, il sera régulé par tout moyen connu par ailleurs de l'homme du métier, de manière à ne pas dépasser 45 mmoles/l/heure.

Conformément au procédé de l'invention, la biomasse extraite du fermenteur est traitée est pour atteindre une pureté supérieure à 95 %, exprimée en poids sec de biomasse sur poids sec total du milieu de fermentation, par tout moyen connu par l'homme du métier.

De manière avantageuse, la société Demanderesse recommande de laver les solubles interstitiels par une succession de concentration (par centrifugation)/dilution de la biomasse, comme il sera exemplifié ci-après.

Cette biomasse ainsi purifiée de ses solubles interstitiels est ensuite ajustée préférentiellement à une matière sèche comprise entre 6 et 12 %, de préférence à une matière sèche comprise entre 10 et 12 % avec de l'eau déminéralisée ou purifiée, de préférence purifiée.

La deuxième étape du procédé conforme à l'invention consiste à traiter la biomasse ainsi obtenue à l'aide d'une enzyme de type protéase choisie dans le groupe des protéases neutres ou basiques, par exemple l'alcalase, de manière à rompre la paroi cellulaire desdites microalgues tout en prévenant la formation de l'émulsion produite par ledit traitement enzymatique.

En préambule de cette étape de lyse enzymatique de la paroi cellulaire, la biomasse à 12 % de matière sèche est placée dans un réacteur équipé avec une hélice marine (peu cisaillant) et chicanes (afin de casser l'effet de vortex produit) de manière à limiter l'émulsification du lysat cellulaire qui sera généré par le traitement enzymatique, tout en permettant un mélange homogène favorisant l'action de l'enzyme lytique.

La température est ajustée à une température supérieure à 50°, de préférence d'environ 60°C, et à un pH supérieur à 7, de préférence d'environ 8. Dans la présente demande, le terme « environ » signifie la valeur indiquée $\pm$ 10% de celle-ci, de préférence $\pm$ 5% de celle-ci. Bien entendu, est compris la valeur exacte. Par exemple, environ 100 signifie entre 90 et 110, de préférence entre 95 et 105.

Ces conditions sont optimales pour l'activité de l'enzyme Alcalase (par exemple celle commercialisée par la société NOVOZYMES) qui est utilisée à une concentration comprise entre 0,4 et 1 % /sec, de préférence 1 % / sec.

La durée de la lyse est comprise entre 2 et 8 h, de préférence 4 h.

A la fin de la lyse, la société Demanderesse recommande d'ajouter de l'éthanol à plus de 5 % (v/v), de préférence d'environ 10 % (v/v) dans le mélange réactionnel (forme émulsion huile dans eau) et de maintenir sous agitation pendant encore 15 minutes supplémentaires.

L'éthanol est ajouté en proportion mineure au système comme agent de déstabilisation de l'émulsion.

La troisième étape du procédé conforme à l'invention consiste à centrifuger le mélange réactionnel ainsi obtenu afin de séparer l'huile de la phase aqueuse.

L'émulsion déstabilisée à l'éthanol obtenue au terme de l'étape précédente est centrifugée.

Trois phases sont obtenues :

- une phase supérieure légère (huile),
- une phase intermédiaire aqueuse majoritaire (eau + hydrosolubles) et
- une phase inférieure (culot de débris cellulaires).

La séparation de ces trois phases est réalisée avec un dispositif de type séparatrice à trois sorties en mode concentrateur, tel la CLARA 20 commercialisée par la société ALPHA LAVAL, qui permet la récupération de la phase supérieure légère (huile) extraite de la phase aqueuse et des débris cellulaires.

La phase aqueuse est extraite quant à elle par la sortie phase lourde de la séparatrice. La phase solide est extraite par autodébourbage.

Le lysat cellulaire obtenu au terme de l'étape 2 du procédé conforme à l'invention peut être chauffé à une température comprise entre 70 et 90°C, notamment entre 70 et 80°C et de préférence de 80°C, puis est alimenté à l'aide d'une pompe volumétrique (pour limiter ici encore l'émulsification). De préférence, son pH peut être amené à une valeur comprise entre 8 et 12, de préférence à une valeur de 10.

La force centrifuge est supérieure à 4000 g, de préférence entre 6000 et 10000 g.

La phase légère non émulsifiée est obtenue de préférence en une seule passe.

La quatrième étape du procédé conforme à l'invention consiste enfin à récupérer la phase supérieure huileuse enrichie en squalène.

L'invention sera mieux comprise à l'aide des exemples qui suivent, lesquels se veulent illustratifs et non limitatifs.

**Exemple 1**

La fermentation des microalgues a été conduite ici en deux phases de préculture successives préalables avant la phase de culture / production proprement dite.

Pour cette expérimentation, l'ajout des vitamines a été assuré dans le premier milieu de préculture, mais a été optionnel dans le second milieu de préculture et en production.

Les milieux de préculture présentaient alors la composition présentée dans les tableaux I et II suivants :

Tableau I

| Milieu de la première préculture | % |
|---|---|
| Glucose | 3 |
| Extraits de levure | 0,4 |
| Sodium glutamic acid | 6,42 |
| NaCl | 1,25 |
| $MgSO_4$ | 0,4 |
| KCl | 0,05 |
| $CaCl_2$ | 0,01 |
| $NaHCO_3$ | 0,05 |
| $KH_2PO_4$ | 0,4 |
| Mélange vitamines | 0,14 |
| Oligo-éléments | 0,8 |

Tableau II

| Milieu de la seconde préculture | % |
|---|---|
| Glucose | 8,57 |
| Sodium glutamic acid | 6,42 |
| Extraits de levure | 0,64 |
| Na Cl | 2 |
| $KH_2PO_4$ | 0,64 |
| $MgSO_4$ | 2,29 |
| $CaCl_2$ | 0,03 |
| $NaHCO_3$ | 0,03 |
| $Na_2SO_4$ | 0,03 |
| Mélange de vitamines | 0,14 |
| Oligo-éléments | 0,2 |

D'une manière générale on a utilisé de l'anti-mousse Clerol « FBA3107 » à 1 ml/l. Eventuellement on a utilisé 50 mg /L de Pénicilline G "sodium salt" afin d'éviter la croissance de bactéries contaminantes. Le glucose a été stérilisé avec le $KH_2PO_4$ et séparément du reste du milieu car on a évité ainsi la formation d'un précipité (Ammonium-Phosphate-Magnésium). Le mélange des vitamines et les oligo-éléments ont été ajoutés après filtration stérilisante. La composition du milieu de culture / production est donné par le tableau III suivant.

Tableau III

| | % |
|---|---|
| Glucose Ajout à T0 | 7,5 |
| Urée | 1 |
| Extraits de levure | 1,2 |
| Na Cl | 0,25 |
| $KH_2PO_4$ | 0,96 |
| $MgSO_4$ | 1,2 |
| $CaCl_2$ | 0,12 |
| $NaHCO_3$ | 0,12 |
| KCL | 0,08 |
| Ajout du mélange de vitamines | 0,4 |
| Oligo-éléments | 0,56 |

La composition des mélanges de vitamines et des oligoéléments est donnée dans les tableaux IV et V suivants :

Tableau IV

| Mélange vitamines | g/L |
|---|---|
| B1 | 45 |
| B6 | 45 |
| B12 | 0.25 |

Tableau V

| Oligo-éléments | g/L |
|---|---|
| $MnCl_2\ 2H_2O$ | 8.60 |
| $CoCl_2\ 6H_2O$ | 0.2 |
| $NiSO_4\ 6H_2O$ | 7.50 |
| $Na_2MoO_4\ 2H_2O$ | 0.15 |
| $ZnSO_4\ 7\ H_2O$ | 5.70 |
| $Cu\ So_4\ 5\ H_2O$ | 6.50 |
| $FeSO_4\ 7\ H_2O$ | 32.00 |
| $ZnCl_2$ | 1.50 |

Conduite de la fermentation

La première préculture a été réalisée en Erlenmeyers de 500 ml munis de baffles, dans lesquels on a ajouté une goutte d'antimousse CLEAROL FBA 3107 commercialisé par la société COGNIS GmbH Düsseldorf.

Le milieu de culture a été filtré après dissolution complète de ses constituants, complété éventuellement avec de la pénicilline G "sodium salt" à raison de 0,25 mg/l.

L'inoculation a été réalisée par prélèvement de colonies de microalgues cultivées en boite de Pétri (à raison d'une oese de 10 $\mu$l).

L'incubation a duré 24 à 36 heures, à une température de 28°C, sous agitation à 100 rpm (sur agitateur orbital).

La biomasse décantant (ou adhérant à la paroi), on a pris bien soin de prélever 3 à 5 ml après avoir bien agité l'Erlenmeyer.

Pour la seconde préculture, on a utilisé des Erlenmeyers de 2 l, muni de baffles et de tuyauterie.

On a ajouté une goutte d'antimousse et l'extrait de levures dans 100 ml d'eau.

L'ensemble des constituants du milieu a été filtré après dissolution dans 300 ml d'eau déminéralisée. On pouvait éventuellement ajouter de la pénicilline G "sodium salt" et au préalable dans l'Erlenmeyer une goutte d'antimousse avant sa stérilisation.

L'ensemencement s'est fait ensuite avec 3 à 5 ml de la première préculture.

L'incubation a été réalisée à 28°C pendant encore 24 à 36 heures, sous agitation à 100 rpm.

La culture proprement dite a été réalisée de la manière suivante en réacteur de 20 l.

- stérilisation du milieu pour partie dans le réacteur, et séparément pour l'autre partie de manière à éviter la formation d'un précipité,
- ensemencement réalisé à partir de la biomasse produite en fin de seconde préculture à raison de 0,5 % v/v du milieu de culture,
- culture maintenue à 30°C
- taux de transfert d'oxygène fixé à 35 - 40 mmoles/l/h,
- aération de 0,2 à 0,3 WM,
- pH initial > 5,5.
- alimentation du glucose dès que la concentration est > 20 %, de manière à maintenir une concentration en glucose comprise entre 15 et 70 g/l.

Le tableau IV suivant présente les résultats obtenus la *Schizochytrium sp.* de la société Demanderesse.

Tableau IV :

| Essais | E |
| --- | --- |
| Température des précultures (°C) | 28 |
| Température de culture (C) | 30 |
| Titre en squalène en fin de culture (g/l) | 4,4 |
| Biomasse (g/l) | 54 |
| g/ 100 g de squalène sur biomasse sèche | 8,2 |

Méthode de quantification du squalène dans la biomasse de *Schizochytrium sp.*

L'analyse a été réalisée par RMN du proton à 25°C après cassage billes de la biomasse et extraction au chloroforme/méthanol à froid. La quantification a été faite au moyen d'un étalon interne tel que décrit ci-dessous.

Les spectres ont été obtenus sur un spectromètre AVANCE III 400 (Bruker Spectrospin), opérant à 400 MHz.

Cassage de la biomasse : Peser précisément environ 200 mg de biomasse fraîche. Ajouter environ 1-1,5 cm de billes verre et 0,1 mL de Méthanol. Fermer hermétiquement le tube et agiter au moyen de l'agitateur vortex pendant au moins 5 min.

Extraction à froid : Ajouter environ 2 mg de triphényl phosphate (TPP), 0,9 mL de Méthanol et 2 mL de chloroforme. Fermer hermétiquement le tube et agiter au moyen de l'agitateur vortex pendant 1 min. Placer au réfrigérateur. Après décantation (au minimum 1 heure), récupérer délicatement la phase supérieure limpide et la transférer dans un godet verre pour évaporation à sec, à température ambiante, sous courant d'azote. Solubiliser l'extrait sec dans 0,5 mL de $CDCl_3$ et 0,1 mL de $CD_3OD$ et transférer dans un tube RMN.

Enregistrement du spectre : Effectuer l'acquisition, sans suppression de solvant, sans rotation, avec un temps de relaxation d'au moins 15 s, après les réglages appropriés de l'instrument. La fenêtre spectrale doit être au moins comprise entre -1 et 9 ppm en calibrant le spectre sur le pic de chloroforme à 7,25 ppm. Le spectre est exploité après transformation de Fourier, correction de phase et soustraction de la ligne de base en mode manuel (sans multiplication exponentielle, LB=GB=0).

Exploitation du signal : Attribuer la valeur 100 au massif du TPP ne contenant pas le signal du chloroforme entre 7,05 et 7,15 ppm (comptant pour 9 protons TPP). Intégrer la surface du signal Squalène à 1,55 ppm (singulet comptant pour 6 protons).

Calcul et expression des résultats : Les résultats ont été exprimés en pourcentage massique brut.

$$\text{Teneur} = \frac{A_s \times P_{TPP}}{6 \times 100} \times \frac{W_{TPP}}{M_{TPP}} \times M_S \times \frac{100}{PE}$$

avec

$A_s$ : surface du signal Squalène à 1,55 ppm.
$P_{TPP}$: nombre de protons du massif TPP intégré : 9
$W_{TPP}$ : masse, en grammes, de TPP pesé
$M_{TPP}$ : masse molaire, en grammes par mole, du TPP ($M_{TPP}$=326 g/mol)
$M_S$ : masse molaire, en grammes par mole, du Squalène ($M_S$ =410 g/mol) PE : masse, en grammes, de biomasse fraîche

## Exemple 2 : Extraction du squalène selon l'invention

La biomasse obtenue au terme de l'exemple 1 était à une concentration de 54 g/l en fin de fermentation.
Le titre en squalène obtenu en fin de fermentation était de 4,4 g/l.
La biomasse extraite du fermenteur et lavée des solubles interstitiels par succession de deux séries de concentration par centrifugation (5 minutes à 5000 g) et dilution de la biomasse (à raison de 1/3 Vculot / Veau).
La concentration cellulaire sèche sur la matière sèche brute totale est de 95 %.
La matière sèche est ensuite ajustée à 12% avec de l'eau distillée.
La biomasse lavée est agitée dans un réacteur labo de type Fermenteur 2 l (tel que ceux commercialisés par la société Interscience) équipé avec une hélice marine et chicanes.
Ce système permet de limiter l'émulsification du lysat cellulaire généré tout en permettant un bon mélange indispensable pour l'action de l'enzyme lytique.
La température est ajustée à 60°C et le pH est régulé à environ 8 avec de la soude.
Ces conditions sont optimales pour l'activité de l'enzyme Alcalase (Novozymes) ajoutée à hauteur de 1%/sec.
La durée de la lyse est fixée à 4 h.
En fin de lyse, on ajoute 10 % d'éthanol ($V_{éthanol}/V_{lysat}$) dans le mélange réactionnel (émulsion huile dans eau) maintenue 15 min supplémentaires sous agitation.
La température est relevée à 80°C et on centrifuge ensuite sur module de centrifugation ALPHA LAVAL CLARA 20, configuré en mode concentrateur à 3 sorties.
Cette configuration est particulièrement bien adaptée pour la séparation d'un mélange triphasique de type solide/liquide/liquide.
La mise en rotation à 9600 tr/min permet d'atteindre environ 10000 g.
L'alimentation en lysat cellulaire est réalisée à l'aide d'une pompe volumétrique à un débit de 100 à 400 l/h.
L'interface entre la phase lourde et la phase légère est déplacée en réglant la contrepression en sortie phase lourde.
La fréquence d'autodébourbage est réglée sur une fréquence de 2 à 15 min. L'huile brute a été ainsi récupérée avec un rendement de plus de 85 % et renferme ainsi la quasi totalité du squalène produit.

## Exemple 3 : Exemple comparatif d'extraction du squalène par procédé classique à l'hexane

Tout comme décrit dans l'exemple 2 :

- La biomasse obtenue au terme de l'exemple 1 était à une concentration de 54 g/l en fin de fermentation.
- Le titre en squalène obtenu en fin de fermentation était de 4,4 g/l.

La biomasse extraite du fermenteur et également concentrée par centrifugation à 120 g/l.
La biomasse a été maintenue sous agitation à 150 rpm dans une cuve 50 l, et est chauffée à 60°C.
Le pH a été alors ajusté à 10 à la potasse 45 %.
Ces conditions ont été maintenues pendant 6 h pour parvenir à une lyse alcaline complète.
La qualité de la lyse a été suivie au microscope optique et par centrifugation d'échantillon (2 min, 10000 g).
En fin de lyse, 10 litres d'éthanol (1 volume d'éthanol /volume lysat) ont été ajoutés dans la cuve maintenue à 45°C et agitée pendant 10 min.

10 litres d'hexane ont été ensuite ajoutés dans la cuve maintenue agitée pendant 30 min.

Le mélange a été ensuite centrifugé afin de séparer la fraction légère (hexane + huile) qui a été stockée dans une cuve de 1 m$^3$.

La phase lourde (aqueuse) a été à nouveau mise en présence de 10 litres d'hexane pour procéder à une seconde extraction sur le même schéma que précédemment afin d'augmenter le rendement d'extraction.

Les deux fractions organiques ont été regroupées afin de procéder à l'évaporation de l'hexane en évaporateur rotatif.

Les résidus d'hexane de l'huile extraite ont été éliminés par évaporation à l'évaporateur à film raclé (80°C ; 1 mbar).

L'huile brute a été ainsi récupérée avec un rendement de 70%.

Ce procédé d'extraction « classique » est donc bien moins efficace que celui conforme à l'invention.

## Revendications

1. Procédé d'extraction sans solvant organique de squalène produit par fermentation de microalgues appartenant à la famille des Thraustochytriales sp., **caractérisé en ce qu'**il comprend les étapes suivantes :

   1) préparer une biomasse de microalgues appartenant à la famille des Thraustochytriales de manière à réduire la concentration en solubles interstitiels et atteindre ainsi une pureté supérieure à 95 % exprimée en poids sec de biomasse sur poids sec total du milieu de fermentation,
   2) traiter la biomasse ainsi obtenue à l'aide d'une enzyme de type protéases choisies dans le groupe des protéases neutres ou basiques de manière à rompre la paroi cellulaire desdites microalgues tout en prévenant la formation de l'émulsion produite par ledit traitement enzymatique,
   3) centrifuger le mélange réactionnel ainsi obtenu afin de séparer l'huile de la phase aqueuse, et
   4) récupérer l'huile brute enrichie en squalène ainsi produite.

2. Procédé selon la revendication 1, **caractérisé en ce que** la biomasse purifiée à l'étape 1) est ensuite ajustée à une matière sèche comprise entre 6 et 12 %, de préférence à une matière sèche comprise entre 10 et 12 %.

3. Procédé selon l'une ou l'autre des revendications 1 et 2, **caractérisé en ce que** le traitement enzymatique de l'étape 2) est effectué sous agitation non cisaillante et peu émulsifiante dans un dispositif muni d'une hélice marine et de chicanes.

4. Procédé selon l'une quelconque des revendications 1 à 3, **caractérisé en ce que** le traitement enzymatique de l'étape 2) est effectué à une température supérieure à 50°C, de préférence de l'ordre de 60°C, et à un pH supérieur à 7, de préférence d'environ 8.

5. Procédé selon l'une quelconque des revendications 1 à 4, **caractérisé par le fait que** de l'éthanol est ajouté en fin de traitement enzymatique à plus de 5 % (v/v), de préférence d'environ 10 % (v/v).

6. Procédé selon la revendication 5, **caractérisé par le fait que** le traitement à l'éthanol est réalisé sous agitation pendant plus de 10 minutes, de préférence pendant 15 minutes.

7. Procédé selon l'une quelconque des revendications 1 à 6, **caractérisé en ce que** le mélange réactionnel est à une température comprise entre 70 et 90°C, de préférence de 80°C, avant la centrifugation.

8. Procédé selon l'une quelconque des revendications 1 à 7, **caractérisé en ce que** le pH du mélange réactionnel est amené à une valeur comprise entre 8 et 12, de préférence à une valeur de 10, avant la centrifugation.

9. Procédé selon l'une quelconque des revendications 1 à 8, **caractérisé en ce que** la centrifugation est réalisée dans une séparatrice à trois sorties en mode concentrateur qui permet la récupération de la phase supérieure légère (huile) extraite de la phase aqueuse et des débris cellulaires.

10. Procédé selon la revendication 9, **caractérisé en ce que** la centrifugation est réalisée avec une force centrifuge supérieure à 4000 g, de préférence entre 6000 et 10000 g, de préférence d'environ 10000 g.

**Patentansprüche**

1. Verfahren zur Extraktion von Squalen ohne organisches Lösungsmittel, hergestellt durch Fermentation von Mikroalgen, die zur Familie der Thraustochytriales sp. gehören, **dadurch gekennzeichnet, dass** es die folgenden Schritte umfasst:

   1) Herstellen einer Biomasse von Mikroalgen, die zur Familie der Thraustochytriales gehören, um die Konzentration an löslichen Interstitiellen zu verringern und so eine Reinheit von mehr als 95% zu erreichen, ausgedrückt als Trockengewicht der Biomasse gegenüber dem Gesamttrockengewicht des Fermentationsmediums,
   2) Behandeln der so erhaltenen Biomasse unter Verwendung eines Enzyms vom Protease-Typ, ausgewählt aus der Gruppe der neutralen oder basischen Proteasen, um die Zellwand der Mikroalgen zu durchbrechen, während die Bildung der durch die enzymatische Behandlung erzeugten Emulsion verhindert wird,
   3) Zentrifugieren des so erhaltenen Reaktionsgemischs, um das Öl von der wässrigen Phase zu trennen, und
   4) Rückgewinnen des so erzeugten Rohöls, das mit Squalen angereichert ist.

2. Verfahren nach Anspruch 1, **dadurch gekennzeichnet, dass** die in Schritt 1) gereinigte Biomasse dann auf eine Trockenmasse zwischen 6 und 12%, vorzugsweise auf eine Trockenmasse zwischen 10 und 12% eingestellt wird.

3. Verfahren nach einem der Ansprüche 1 und 2, **dadurch gekennzeichnet, dass** die enzymatische Behandlung von Schritt 2) unter nicht scherendem und wenig emulgierendem Rühren in einer Vorrichtung durchgeführt wird, die mit einem Schiffspropeller und Leitblechen versehen ist.

4. Verfahren nach einem der Ansprüche 1 bis 3, **dadurch gekennzeichnet, dass** die enzymatische Behandlung von Schritt 2) bei einer Temperatur von mehr als 50 °C, vorzugsweise in der Größenordnung von 60 °C, und bei einem pH von mehr als 7, vorzugsweise etwa 8, durchgeführt wird.

5. Verfahren nach einem der Ansprüche 1 bis 4, **dadurch gekennzeichnet, dass** Ethanol am Ende der enzymatischen Behandlung mit mehr als 5% (v/v), vorzugsweise etwa 10% (v/v) zugegeben wird.

6. Verfahren nach Anspruch 5, **dadurch gekennzeichnet, dass** die Behandlung mit Ethanol unter Rühren während mehr als 10 Minuten, vorzugsweise während 15 Minuten durchgeführt wird.

7. Verfahren nach einem der Ansprüche 1 bis 6, **dadurch gekennzeichnet, dass** das Reaktionsgemisch vor dem Zentrifugieren eine Temperatur zwischen 70 und 90 °C, vorzugsweise 80 °C aufweist.

8. Verfahren nach einem der Ansprüche 1 bis 7, **dadurch gekennzeichnet, dass** der pH des Reaktionsgemischs vor dem Zentrifugieren auf einen Wert zwischen 8 und 12, vorzugsweise auf einen Wert von 10 gebracht wird.

9. Verfahren nach einem der Ansprüche 1 bis 8, **dadurch gekennzeichnet, dass** das Zentrifugieren in einem Separator mit drei Ausgängen im Konzentratormodus durchgeführt wird, der die Rückgewinnung der leichten oberen Phase (Öl), die aus der wässrigen Phase und aus den Zelltrümmern extrahiert ist, ermöglicht.

10. Verfahren nach Anspruch 9, **dadurch gekennzeichnet, dass** das Zentrifugieren mit einer Zentrifugalkraft von mehr als 4000 g, vorzugsweise zwischen 6000 und 10000 g, vorzugsweise etwa 10000 g durchgeführt wird.

**Claims**

1. Method for extracting, without organic solvent, squalene produced by fermenting microalgae belonging to the *Thraustochytriales sp.* family, **characterised in that** it comprises the following steps:

   1) preparing a biomass of microalgae belonging to the *Thraustochytriales* family so as to reduce the concentration of interstitial soluble matter and thus to achieve a purity greater than 95% expressed as the dry weight of biomass over the total dry weight of the fermentation medium,
   2) treating the biomass thus obtained by means of an enzyme of the protease type chosen from the group of neutral or basic proteases so as to break the cell wall of said microalgae while preventing the formation of emulsion produced by said enzymatic treatment,
   3) centrifuging the reaction mixture thus obtained in order to separate the oil from the aqueous phase, and

4) recovering the squalene-enriched crude oil thus produced.

2. Method according to claim 1, **characterised in that** the biomass purified at step 1) is then adjusted to a dry matter content of between 6 and 12%, preferably to a dry matter content of between 10 and 12%.

3. Method according to claims 1 or 2, **characterised in that** the enzymatic treatment of step 2) is carried out with non-shearing and weakly-emulsifying stirring in a device equipped with a propeller stirrer and baffles.

4. Method according to any one of claims 1 to 3, **characterised in that** the enzymatic treatment of step 2) is carried out at a temperature above 50°C, preferably around 60°C, and at a pH above 7, preferably approximately 8.

5. Method according to any one of claims 1 to 4, **characterised in that** ethanol is added at the end of enzymatic treatment at more than 5% (v/v), preferably approximately 10% (v/v).

6. Method according to claim 5, **characterised in that** the ethanol treatment is carried out with stirring for more than 10 minutes, preferably for 15 minutes.

7. Method according to any one of claims 1 to 6, **characterised in that** the reaction mixture is at a temperature of between 70 and 90°C, preferably 80°C, before centrifugation.

8. Method according to any one of claims 1 to 7, **characterised in that** the pH of the reaction mixture is brought to a value of between 8 and 12, preferably to a value of 10, before centrifugation.

9. Method according to any one of claims 1 to 8, **characterised in that** the centrifugation is carried out in a three-output separator in concentrator mode which allows the recovery of the light upper phase (oil) extracted from the aqueous phase and cell debris.

10. Method according to claim 9, **characterised in that** the centrifugation is carried out with a centrifugal force of greater than 4000 g, preferably between 6000 and 10,000 g, preferably approximately 10,000 g.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010023551 A
- KR 20080017960
- EP 1252324 A
- EP 1305440 A

**Littérature non-brevet citée dans la description**

- **BHATTACHARJEE, P. et al.** *World J. Microb. Biotechnol.,* 2001, vol. 17, 811-816
- **LEWIS et al.** *Mar. Biotechnol.,* 2001, 439-447
- **YUE JIANG et al.** *J. Agric. Food Chem.,* 2004, vol. 52, 1196-1200
- **QIAN LI et al.** *J. Agric. Food Chem.,* 2009, vol. 57, 4267-4272
- **LEWIS et al.** *Mar. Biotechnol.,* 2001, vol. 3, 439-447
- **G. CHEN et al.** *New Biotechnology,* 2010, vol. 27-4, 382-389
- **K. W. FAN et al.** *World J. Microbiol. Biotechnol.,* 2010, vol. 26-3, 1303-1309
- **C-J YUE ; Y. JIANG.** *Process Biochemisty,* 2009, vol. 44, 923-927
- **YUE JIANG et al.** *J. Agric. Food Chem.,* 2004, vol. 52, 1196-1200
- **C-J YUE.** *Y. JIANG, Process Biochemisty,* 2009, vol. 44, 923-927
- **BENEMANN, J. ; OSWALD, W.** Systems and Economic Analysis of Microalgae Ponds for Conversion of CO2 to Biomass. *Report prepared for the Pittsburgh Energy Technology Center under Grant No. DE-FG22-93PC93204,* 1996
- **G. CHEN.** *New Biotechnology,* 2010, vol. 27-4, 382-389